# EUROPÄISCHE PATENTANMELDUNG

(11) **EP 3 904 440 A1**
(43) Veröffentlichungstag der Anmeldung: **03.11.2021**
(21) Anmeldenummer: 21173219.3
(22) Anmeldetag: 22.08.2016
(51) Int. Cl.: C08K 3/22, C08L 67/02, A43B 17/14, C08J 3/21, A61N 5/06, D01D 1/06, D01D 5/00, D01F 1/10, A43B 1/00, A43B 7/00, A43B 23/02, A61K 33/26, B29C 48/14, B29C 48/295, C09K 11/02, D01F 6/60, D01F 6/62, A61P 7/00, B29C 48/05, B29C 48/08, C09K 11/60

(54) **POLYMERMATERIAL UMFASSEND EIN ODER MEHRERE UNTERSCHIEDLICHE DOTIERUNGSELEMENTE, VERWENDUNG UND HERSTELLUNGSVERFAHREN**

(62) Teilanmeldung aus: 16757622.2
(71) Anmelder: S-Techs GmbH, 22393 Hamburg (DE)
(72) Erfinder: van Hattum, Edgar-Johannes, 29303 Bergen (DE)
(74) Vertreter: Pfleiderer, Bernhard

(57) **Zusammenfassung**

Die Erfindung betrifft ein Polymermaterial umfassend ein oder mehrere unterschiedliche Dotierungselemente, dadurch dass das oder mindestens eines der unterschiedlichen Dotierungselemente, zumindest teilweise eine vom menschlichen oder tierischen Körper emittierte elektromagnetische Strahlung absorbieren und zumindest teilweise eine elektromagnetische Strahlung im Infrarot Bereich, vorzugsweise Infrarot C Bereich emittieren sowie ein textiles Material umfassend das erfindungsgemäße Polymermaterial. Des Weiteren betrifft die Erfindung medizinische und nicht-medizinische Verwendungen des erfindungsgemäßen Polymermaterials und ein Herstellverfahren des erfindungsgemäßen Polymermaterials.

## Beschreibung

### TECHNISCHES FELD DER ERFINDUNG

Die Erfindung betrifft ein Polymermaterial umfassend ein oder mehrere unterschiedliche Dotierungselemente, dadurch dass das oder mindestens eines der unterschiedlichen Dotierungselemente, zumindest teilweise eine vom menschlichen oder tierischen Körper emittierte elektromagnetische Strahlung absorbieren und zumindest teilweise eine elektromagnetische Strahlung im Infrarot Bereich, insbesondere Infrarot C Bereich emittieren sowie ein textiles Material umfassend das erfindungsgemäße Polymermaterial. Des Weiteren betrifft die Erfindung medizinische und nicht-medizinische Anwendungen des erfindungsgemäßen Polymermaterials und ein Herstellverfahren des erfindungsgemäßen Polymermaterials.

### HINTERGRUND DER ERFINDUNG

Stoffwechseladäquate Transportvorgänge, die im Gewebe durch Stoffaustausch zwischen dem Blut und den Gewebezellen erfolgen, sind Voraussetzung dafür, dass physiologische Organfunktionen erhalten oder wiederhergestellt werden können. Physiologische Organfunktionen sind wiederum Voraussetzung für eine adäquate körperliche und geistige Leistungsfähigkeit eines menschlichen oder tierischen Organismus. Hierbei kommt der Mikrozirkulation des Blutes, d.h. die Perfusionszustände in den Kapillaren, Arteriolen und Venolen, eine wichtige Funktion zu, da dort die Transportphänomene des Stoffaustausches mit den Gewebezellen, d.h. die Gewebenutrition, und die ersten Schritte der Immunreaktion stattfinden (Klopp et al., "Änderung des Funktionszustandes der Mikrozirkulation durch eine adjuvante BioKorrektur-Behandlung bei Patienten mit Diabetes Mellitus Typ II, Archiv Euromedica, 2014, Vol. 4, Num. 1, Seite 36).

An die sich ändernden Stoffwechselbedürfnisse der Organe ist eine Durchblutungsregulation dann optimal angepasst, wenn sie ausreichend rasche Strömungsgeschwindigkeiten und eine stoffwechseladäquate Verteilung des Plasma-Blutzell-Gemisches in den mikrovaskulären Netzwerken gestattet. Im Mittelpunkt steht dabei die Endothel-vermittelte schubspannungsabhängige Tonusregulation der großkalibrigen und kleinkalibrigen Arteriolen und ihrer damit verbundenen vasomotorischen Aktivitäten. Hauptkriterien für den Funktionszustand sind somit Strömungsgeschwindigkeiten, Strömungsfluss und Verteilungszustand des Plasma-Blutzell-Gemisches in den Mikrogefäßen, welche durch die Entmischungsphänomene von Plasma und Blutzellen determiniert werden. Hierdurch werden nicht nur die Mechanismen der Temperaturregulation, sondern vor allem die Gewebenutrition und die Immunabwehr bedarfsgerecht gestaltet. Ausdruck des Funktionszustandes ist die jeweilige Sauerstoffausschöpfung des Blutes. Somit gilt: beeinflusst man die Mikrozirkulation physiologisch vorteilhaft, dient dies einer optimalen Zell- und Organfunktion und einer wirksamen körpereigenen Immunabwehr.

Störungen oder defizitäre Funktionszustände der Mikrozirkulation beeinträchtigen die Organfunktion und führen letztlich zu organischen Schädigungen und einer erhöhten Infektanfälligkeit, zu Wundheilungsstörungen, Ulcerationen etc. Liegen bereits Vorschädigungen vor, wie z.B. bei der diabetischen Mikroangiopathie oder bei sogenannten peripheren Durchblutungsstörungen, so äußern sich organische Schädigungen meist zuerst an den sogenannten Prädestinationsstellen für Gewebeschädigungen. Dies betrifft vor allem oberflächennahe Körperregionen mit kleinen Krümmungsradien, beispielsweise Zehen, Knöchel etc. Ferner ist auch daran zu denken, dass Restriktionen der Mikroperfusion zumindest mitbeteiligt sind, wenn im Knochen-Bänder-Gelenk-System vorzeitig Belastungs- und Überlastungsschäden auftreten, wie beispielsweise Ermüdungsbrüche.

Daraus ist zu folgern, dass jede Maßnahme, die zur Verbesserung des Funktionszustandes der Mikrozirkulation des Blutes führt, prophylaktisch bzw. adjuvant wirksam ist.

Ein Beispiel für die Verbesserung des Funktionszustandes der Mikrozirkulation stellt die adjuvante Bewegungsbehandlung namens "BioKorrektur" dar. In einer placebokontrollierten Untersuchungsreihe wurde herausgefunden, dass die Bewegungsbehandlung namens "BioKorrektur" mit definierter Laufbandbelastung für Patienten mit *Diabetes mellitus* Typ II eine stoffwechseladäquate Mikrozirkulation des Blutes stimuliert (Klopp *et al., ibid.*) und damit adjuvant wirksam ist.

Gerade vor dem Hintergrund, dass einige Patienten aufgrund ihrer Erkrankung und damit körperlichen Konstitution nur eine sehr eingeschränkte Bewegungsfähigkeit besitzen, besteht das Bedürfnis eine alternative Möglichkeit bereit zu stellen, die Mikrozirkulation des Blutes in einem stoffwechseladäquat zu stimulieren.

### ZUSAMMENFASSUNG DER ERFINDUNG

Die vorgenannte Aufgabe wird anhand der erfindungsgemäß beanspruchten Gegenstände gelöst. Vorteilhafte Ausgestaltungen werden in den abhängigen Ansprüchen sowie in der nachfolgenden Beschreibung dargestellt.

Dementsprechend betrifft ein erster Erfindungsgegenstand der vorliegenden Erfindung ein Polymermaterial umfassend ein oder mehrere unterschiedliche Dotierungselemente, dadurch gekennzeichnet, dass das oder mindestens eines der unterschiedlichen Dotierungselemente zumindest teilweise eine vom Wirbeltier, vorzugsweise Menschen emittierte elektromagnetische Strahlung absorbieren und zumindest teilweise eine elektromagnetische Strahlung im Infrarot Bereich emittieren.

Ein zweiter Erfindungsgegenstand der vorliegenden Erfindung betrifft ein textiles Material umfassend ein erfindungsgemäßes Polymermaterial.

Ein dritter Erfindungsgegenstand der vorliegenden Erfindung betrifft ein erfindungsgemäßes Polymermaterial zur Verwendung als Adjuvanz oder Prophylaktikum in therapeutischen Verfahren.

Ein vierter Erfindungsgegenstand der vorliegenden Erfindung betrifft ein erfindungsgemäßes Polymermaterial zur Verwendung bei der prophylaktischen oder adjuvanten Behandlung einer stoffwechselinadäquaten Mikrozirkulation des Blutes im Wirbeltier, vorzugsweise Menschen; bei der Behandlung von nekrotischen Gefäßen; bei der Wundheilung, vorzugsweise bei Wundbehandlung bei Dekubitus, Diabetisches Fußsyndrom, Ulcus cruris, Verbrennungen und für die Wundbehandlung von vielen weiteren chronischen und sekundär heilenden Wunden; von Diabetes mellitus, insbesondere Diabetes mellitus Typ I und/oder Diabetes mellitus Typ II; von Krebserkrankungen; von Protein-bedingten Erkrankungen, beispielsweise Alzheimer Erkrankung, oder Demenzerkrankung; von Thrombozytenerkrankungen; von Erythrozytenerkrankungen; von immunologischen Erkrankungen, wie beispielsweise immunologische Hyperaktivität; von infektiösen Erkrankungen, beispielsweise Wundinfektionen; von neurologischen Erkrankungen, insbesondere soweit diese auf einer Erkrankung der Ummantelung der Nervenzellen und/oder Synapsen beruhen.

Ein fünfter Erfindungsgegenstand der vorliegenden Erfindung betrifft eine Verwendung eines erfindungsgemäßen Polymermaterials zur nicht-medizinischen, insbesondere sportlichen Leistungssteigerung eines Wirbeltiers, vorzugsweise eines Menschen.

Ein sechster Erfindungsgegenstand der vorliegenden Erfindung betrifft ein Verfahren zur Herstellung eines erfindungsgemäßen Polymermaterials, dadurch gekennzeichnet, dass das Verfahren folgende Schritte umfasst oder daraus besteht:
a. Bereitstellung eines geeigneten gelösten Polymers,
b. Bereitstellung eines geeigneten Dotierungselementes,
c. Verdampfen des in Schritt b) bereitgestellten Dotierungselementes mittels geeigneter Verfahren und Einbringen des verdampften Dotierungselementes in das in Schritt a) bereit gestellte gelöste Polymer und
d. Extrudieren des erfindungsgemäßen Polymermaterials im elektrischen Feld.

Die vorstehend beschriebenen Erfindungsgegenstände können, sofern aus Sicht eines Fachmannes sinnvoll, jede mögliche Kombination der bevorzugten erfindungsgemäßen Ausgestaltungen, die nachfolgend und insbesondere auch in den abhängigen Ansprüchen offenbart werden, aufweisen.

### FIGURENBESCHREIBUNG

**Fig. 1** zeigt ein Diagramm umfassend Meßdaten zum Merkmal mittlerer Strömungsfluss der roten Blutzellen in den mikrovaskulären Netzwerken der Skelettmuskulatur Q_{RBC} für Kontrolle und Verum.
**Fig. 2** zeigt ein Diagramm umfassend Meßdaten zum Merkmal Anzahl der blutzellperfundierten Knotenpunkte im Mikrogefäßnetzwerk nNP für Kontrolle und Verum.
**Fig. 3** zeigt ein Diagramm umfassend Meßdaten zum Merkmal venolenseitige Sauerstoffausschöpfung in den mikrovaskularen Netzwerken der Skelettmuskulatur ΔPO₂ für Kontrolle und Verum.
**Fig. 4** zeigt ein Säulendiagramm umfassend Differenzbeträge der Merkmaländerungen zur venolenseitigen Sauerstoffausschöpfung ΔPO₂ in den mikrovaskularen Netzwerken der Skelettmuskulatur der Ausgangswerte am 0. Tag zu den Meßwerten am 30. Tag für Kontrolle und Verum.
**Fig. 5A** **und** **5B** zeigen vitalmikroskopische Befundbeispiele eines älteren Diabetiker (Teilstichprobe D), der Verum-Gruppe, vor Durchführung der Untersuchung (Fig. 5A) und nach Anwendung der erfindungsgemäßen Schuheinlage am 30. Tag der Untersuchung (5B).

### DETAILLIERTE BESCHREIBUNG DER ERFINDUNG

Die vorliegende Erfindung beruht auf der überraschenden Erkenntnis, dass das erfindungsgemäße Polymermaterial bei Körperkontakt mit dem Wirbeltier, vorzugsweise Menschen die Mikrozirkulation des Blutes stoffwechseladäquat stimuliert und dadurch Zell- und Gewebefunktionen sowie die Immunabwehr verbessert.

Nach derzeitigem Kenntnisstand geht man davon aus, dass das erfindungsgemäße Polymermaterial umfassend die ein oder mehreren Dotierungselemente durch die vom Wirbeltier, vorzugsweise Menschen emittierte Strahlung so angeregt wird, dass eine Materialbewegung der Dotierungselemente im erfindungsgemäßen Polymermaterial entsteht. Die dabei entstehende kinetische Energie ruft eine sogenannte Trägerwelle des erfindungsgemäßen Polymermaterials hervor.

Gleichzeitig regt die vom Wirbeltier, vorzugsweise Menschen emittierte Strahlung das erfindungsgemäße Polymermaterial so an, dass zumindest teilweise elektromagnetische Strahlung im Infrarot Bereich, vorzugsweise im Infrarot C Bereich, weiter bevorzugt im Wellenlängenbereich von 3 µm bis 15 µm, weiter bevorzugt im Bereich von 4 bis 12 µm von dem erfindungsgemäßen Polymermaterial emittiert wird.

Derzeit wird davon ausgegangen, dass die kinetische Energie der hervorgerufenen Trägerwelle die zugleich emittierte elektromagnetischen Strahlung im Infrarot Bereich so moduliert, dass die Infrarot Strahlung tiefer in das Gewebe des Wirbeltiers, vorzugsweise Menschen eindringen kann und so die Mirkozirkulation im peripheren Gewebe vorteilhaft beeinflusst. Hierbei korrelieren die Frequenz- und Amplitudenmodulation in der Weise, dass eine Trägerwelle im Bereich von mehr als 60 Dezibel (Db), vorzugsweise 80 bis 130 Db, weiter bevorzugt 86 bis 120 Db die Frequenz der vom erfindungsgemäßen Polymermaterial emittierten Infrarot-Strahlung (im Folgenden auch Infrarot-Welle genannt), insbesondere Infrarot C Strahlung, so dass sich die Amplitude der Infrarot-Welle vergrößert. Mit anderen Worten, die Infrarot-Welle wird durch die Trägerwelle beschleunigt, um entsprechend tiefer in das Gewebe des Wirbeltiers, vorzugsweise Menschen einzudringen.

Damit das erfindungsgemäße Polymermaterial eine ausreichende Menge an vom Körper des Wirbeltiers, vorzugsweise Menschen emittierte elektromagnetische Strahlung absorbiert und die vom erfindungsgemäßen Polymer emittierte Infrarot-Welle entsprechend tief in das Gewebe des Wirbeltiers, vorzugsweise Menschen penetrieren kann, sollte es in geeignetem körpernahmen Abstand angeordnet werden. Vorzugsweise wird das erfindungsgemäße Polymer in einem Abstand von 0 bis 5 cm von der Haut des Wirbeltiers, vorzugsweise Menschen angeordnet, weiter bevorzugt bis 3 cm, noch weiter bevorzugt bis 2 cm, noch weiter bevorzugt bis 1 cm. Sofern in dem erfindungsgemäßen textilen Material, wie zum Beispiel einer Einlegesohle, ein abdeckendes Material zwischen Körper und erfindungsgemäßem Polymermaterial angeordnet ist, kann es notwendig sein dieses so zu durchbrechen, beispielsweise zu lochen, dass die vom Körper des Wirbeltiers, vorzugsweise Menschen emittierte Strahlung vom erfindungsgemäßen Polymermaterial absorbiert werden kann und die vom erfindungsgemäßen Material emittierte Infrarot-Welle in geeigneter Weise in den Körper penetrieren kann.

Insofern unterscheidet sich die vorliegende Erfindung maßgeblich von herkömmlicher Bestrahlung eines Wirbeltiers, insbesondere Menschen mit Infrarot-Wärmelampen, da diese emittierte Infrarot Strahlung nicht so tief in das Gewebe eindringen kann.

Die vorteilhafte Wirkung des erfindungsgemäßen Polymermaterials, insbesondere eines erfindungsgemäßen textilen Materials auf die Mikrozirkulation des Blutes im Wirbeltier, vorzugsweise Menschen wurde anhand einer placebokontrollierten Studie an erfindungsgemäßen Schuheinlagen vs. Kontroll-Schuheinlagen nachgewiesen (vgl. detaillierte Beschreibung im Ausführungsbeispiel C nachfolgend).

Es konnte in dieser Studie nachgewiesen werden, dass einerseits der mittlere Strömungsfluss der roten Blutzellen in den mikrovaskulären Netzwerken der Skelettmuskulatur Q_{RBC} für die erfindungsgemäße Schuheinlage (Verum) gegenüber der Kontrollschuheinlage signifikant verbessert werden konnte. Dies bedeutet, dass ein ausreichendes Druckgefälle zwischen Arteriolen und Venolen als Voraussetzung für einen bedarfsgerechten Strömungsfluss des Plasma-Blutzell-Gemisches in der Mikrozirkulation erreicht wurde. Durch die Verbesserung des mittleren Strömungsflusses der roten Blutzellen in den mikrovaskulären Netzwerken der Skelettmuskulatur Q_{RBC} wird die Anpassungsbreite von Q_{RBC} an sich ändernde Stoffwechselbedürfnisse des zu versorgenden Gewebes verbessert.

Zudem wurde auch die Anzahl der blutzellperfundierten Knotenpunkte im Mikrogefäßnetzwerk nNP für die erfindungsgemäße Schuheinlage (Verum) gegenüber der Kontrollschuheinlage signifikant erhöht. Dies bedeutet, dass eine entsprechend bedarfsgerechte Verteilung des Plasma-Blutzell-Gemisches in den kapillaren Netzwerken erreicht wird. Hierbei verringern sich die Diffusionswege bei gleichzeitig hoher mikrozirkulatorischen Reserven zur Anpassung von nNP an geänderte Aktivitätszustände der zu versorgenden Organe.

Des Weiteren konnte in der Studie gezeigt werden, dass die venolenseitige Sauerstoffausschöpfung in den mikrovaskularen Netzwerken der Skelettmuskulatur ΔPO₂ für die erfindungsgemäße Schuheinlage (Verum) gegenüber der Kontrollschuheinlage signifikant verbessert wurde. Eine hohe Sauerstoffausschöpfung ΔPO₂ führt bei vorliegend verbessertem Strömungsfluss Q_{RBC} zu einem verbesserten Antransport von Substraten für zelluläre Reaktionen, wie Sauerstoff, Nährstoffe, Aminosäuren, Proteine, pharmazeutische Wirkstoffe, plasmatische und zelluläre Faktoren der Immunabwehr und des Glukosestoffwechsels, etc und Abtransport der Stoffwechselprodukte, wie CO, CO₂.

Aufgrund einer erfindungsgemäß verbesserten Mikrozirkulation wird einerseits eine verbesserte Versorgung der Zellen bzw. der Geweberegion beispielsweise mit Sauerstoff, Nährstoffen, Aminosäuren, Proteinen und pharmazeutischen Wirkstoffen erreicht, was die Zell- und damit auch die Organfunktionen verbessert. Aufgrund der verbesserten Zell- und Organfunktionen wird der Körper in der Folge auch leistungsfähiger, was sich sowohl bei der Bewältigung von Krankheiten und Wunden, aber auch bei körperlich anspruchsvollen Tätigkeiten, insbesondere entsprechenden Sportarten positiv auswirkt.

Andererseits kann durch den verbesserten Transport plasmatischer und zellulärer Faktoren der Immunabwehr eine verbesserte Immunabwehr erreicht werden. Dies ist insbesondere vorteilhaft bei der Behandlung von Wunden, um das Infektionsrisiko zu reduzieren.

Das Infektionsrisiko kann zudem durch die von dem erfindungsgemäßen Polymermaterial emittierte modulierte Infrarot Strahlung, vorzugweise Infrarot C Strahlung insbesondere im Bereich von 3 bis 15 µm, reduziert werden, da auch diese keimreduzierend wirkt. Diese Wirkung beruht maßgeblich darauf, dass die emittierte modulierte Infrarot Strahlung opaque zum Abosorptionsspektrum von Wasser liegt, mit anderen Worten trout zum Wasserabsorptionsspektrum ist.

Wie oben bereits beschrieben, geht man derzeit davon aus, dass die verbesserte Mikrozirkulation aufgrund der Anwendung eines erfindungsgemäßen Polymermaterials wohl darauf beruht, dass das erfindungsgemäße Polymermaterial durch die vom Körper emittierte elektromagnetische Strahlung so angeregt wird, dass aufgrund der Materialbewegung der Dotierungselemente im Polymermaterial eine kinetische Energie entsteht, die eine sogenannte Trägerwelle hervorruft und gleichzeitig diese Trägerwelle auf die vom erfindungsgemäßen Polymermaterial emittierte Infrarot Strahlung einen Einfluss hat und diese insbesondere so moduliert, dass diese tiefer in die Gewebe eindringen kann und in den peripheren Blutgefäßen, insbesondere Arteriolen, Kapillaren und Venolen, zu einer Optimierung der Sauerstoffbindung der Hämproteine führt. Hämproteine, wie Hämoglobin, Myoglobin und Cytochrome, gehören zu den wichtigsten physiologischen eisenhaltigen Verbindungen, wobei die Hämproteine im "nicht-Sauerstoff-beladenen" Zustand Eisen (II) Komplexe umfassen, sogenannte Eisenporphyrin-haltige prosthetische Gruppen, (DGE/+GE/SGE/SVE, 2000; Elmadfa und Leitzmann, 1990; Yip, 2001).

Durch die emittierte Infrarot Strahlung, vorzugsweise Infrarot C Strahlung, wird der Eisen-(II)-Komplex in der molekularen Anordnung so geändert, insbesondere "gedehnt" wird, dass er in einer sphärischen Form vorliegt, die sich reaktionsfreudiger gegenüber einer Oxygenierung zum sauerstoffhaltigen Eisen-(III)-Komplex zeigt. Hierbei werden die Oxidationsbrücken zwischen den Fe₂O₃ aufgehoben oder soweit herabgesetzt, dass eine entsprechende Konformationsänderung des Eisen-(II)-Komplexes stattfindet.

Ein Hämprotein, insbesondere Hämoglobin, das sogenannte "gedehnte" Eisen-(II)-Komplexe umfasst, kann zwischen 4% bis 18% mehr Sauerstoff binden und somit mehr Sauerstoff den Zellen und Geweberegionen zur Verfügung stellen. Dieser Vorteil wird insbesondere durch das Merkmal venolenseitige Sauerstoffausschöpfung in den mikrovaskularen Netzwerken der Skelettmuskulatur ΔPO₂ in der placebokontrollierten Studie sowie das Merkmal relative Hämoglobinsättigung rHb (vgl. Ausführungsbeispiel C) belegt.

Zusätzlich geht man davon aus, dass der Proteinkomplex Ferritin, der als Eisendepot bzw. als Transportform von Eisen (III) im Körper fungiert, durch die erfindungsgemäß emittierte Infrarot-C Strahlung dahingehend stimuliert wird mehr Eisen (II) freizusetzen. Die erhöhte Eisen (II) Konzentration führt dazu, dass mehr Eisen (II) in den Hämproteinen gebunden werden kann und so zu einer Erhöhung des Sauerstofftransportes in die Zellen und Gewebe bzw. einen Abtransport von CO₂ aus dem Gewebe ermöglicht.

Der erste Erfindungsgegenstand betrifft demnach ein Polymermaterial umfassend ein oder mehrere unterschiedliche Dotierungselemente, dadurch gekennzeichnet, dass das oder mindestens eines der unterschiedlichen Dotierungselemente zumindest teilweise eine vom Wirbeltier, vorzugsweise Menschen emittierte elektromagnetische Strahlung absorbieren und zumindest teilweise eine elektromagnetische Strahlung im Infrarot Bereich emittieren.

Elektromagnetische Strahlung im Infrarot Bereich umfasst den Spektralbereich zwischen 10⁻³m und 7,8×10⁻⁷ m (1 mm und 780 nm), was einem Frequenzbereich von 3×10¹¹ Hz bis ca. 4×10¹⁴ Hz (300 GHz bis 400 THz) entspricht. Der Infrarot Bereich selbst wird noch einmal in die Bereiche nahes Infrarot (NIR), nämlich Infrarot A (780 nm bis 1.400 nm) und Infrarot B (1.400 nm bis 3.000 nm) und mittleres (MIR) und fernes (FIR) Infrarot, nämlich Infrarot C (MIR: 3.000 nm bis 50 µm, FIR: 50 µm bis 1 mm) unterteilt. Vorzugsweise emittiert das erfindungsgemäße Polymermaterial gemäß aller erfindungsgemäßen Ausgestaltungen elektromagnetische Strahlung im Infrarot C Bereich, vorzugsweise im Bereich von 3 µm bis 50 µm, noch weiter bevorzugt im Bereich von 3 µm bis 20 µm, alternativ von 4 µm bis 15 µm, alternativ von 5 µm bis 12 µm.

Erfindungsgemäß kann jedes für ein textiles Material zu verwendende Polymer als Träger für das erfindungsgemäße Polymermaterial des ersten Erfindungsgegenstandes verwendet werden. Vorzugsweise kennzeichnet sich das Polymermaterial gemäß erstem Erfindungsgegenstand dadurch, dass das Polymer ausgewählt wird aus der Liste bestehend aus der Gruppe der Polyester, beispielsweise Polyethylenterephthalat (PET), der Gruppe der Polyamide, beispielsweise Poly(*p*-phenylenterephthalamid) (PPTA) und Poly(m-phenylenisophthalamid) (PMPI), weiter bevorzugt aus Polyethylenterephthalat (PET).

In einer weiteren kumulativ oder alternativ bevorzugten Ausgestaltung zeichnet sich das erfindungsgemäße Polymermaterial dadurch aus, dass das oder mindestens eines der unterschiedlichen Dotierungselemente eine Eisen Legierung umfasst, bevorzugt eine Eisenoxid-Legierung (Ferrite), weiter bevorzugt eine Erdalkali-Eisenoxid-Legierung (Erdalkali-Ferrite), insbesondere Barium oder Strontium Ferrite, besonders bevorzugt eine SrFe₁₂O₁₉-Legierung (SrO(y Fe₂O₃)₆).

Von der vorliegenden Erfindung sind vorzugsweise in allen Erfindungsgegenständen die folgenden Legierungen vom Schutz ausgenommen: MnFe-Phosphorverbindungen, vorzugsweise MnFe(As,P_{w}GeₓSi_{z})ₛ, insbesondere wobei x = 0,3 - 0,7 und /oder w kleiner gleich 1 - x und z = 1 - x - w und oder FeMn Phosphor-Verbindungen mit As, Si-Phosphor Substitution und ggf. kombiniert mit La(FeMnP)AICo; Legierung mit FeMnP_{0,7}Ge_{0,3}; Legierung mit FeMnP_{0,5}Ge_{0,5}; Legierung mit Fe_{0,86}Mn_{1,14}P_{0,5}Si_{0,35}Ge_{0,15}; und/oder Legierungen mit MnZn.

Erfindungsgemäß sind die Dotierungselemente in den Polymerträger des erfindungsgemäßen Polymermaterials in einer ausreichenden Menge eingebracht, dass die emittierte Infrarot-Welle die Mikrozirkulations positiv beeinflusst. Vorzugsweise haben die Dotierungselemente zum Polymer des erfindungsgemäßen Polymermaterial ein Gewichtsverhältnis von 1:9 bis 9:1, weiter bevorzugt 2:8 bis 8:1, weiter bevorzugt 3:7 bis 7:3, noch weiter bevorzugt, 4:6 bis 6:4, alternativ 1:1. Hierbei wird davon ausgegangen, dass 1 L Polymerlösung 1 kg entspricht. Vorzugsweise umfasst das erfindungsgemäße Polymermaterial gewichtsmäßig weniger Dotierungselement als Polymer, <1:1, vorzugsweise 4:6 Gewichtsteile.

In einer weiteren alternativ oder kumulativ bevorzugten Ausgestaltung kennzeichnet sich das erfindungsgemäße Polymermaterial dadurch, dass das oder die unterschiedlichen Dotierungselemente in das Innere des Polymermaterials eingebracht sind und das Polymermaterial die Dotierungselemente zur Polymermaterialoberfläche isoliert. Mit anderen Worten, das oder die Dotierungselemente sind erfindungsgemäß nicht an der Oberfläche des Polymermaterials angeordnet. Diese Isolierung zur Oberfläche des erfindungsgemäßen Polymermaterials kann insbesondere durch das erfindungsgemäß zu verwendende E-Spinning Verfahren hergestellt werden. Unter Zug und Last des elektrischen Feldes werden die ursprünglich außen angeordneten Dotierungselemente sowohl durch Oxidation als auch mechanisch durch Adhäsion aus der Oberfläche des erfindungsgemäßen Polymermaterials entfernt bzw. sehr stark reduziert.

In einer weiteren alternativ oder kumulativ bevorzugten Ausgestaltung kennzeichnet sich das erfindungsgemäße Polymermaterial dadurch, dass das oder die Dotierungselemente inhomogen in den Polymerträger eingebracht sind. Der Erfinder geht derzeit davon aus, dass eine inhomogene Verteilung des Dotierungselementes eine weitere Verbesserung der Trägerwelle bzw. der emittierten Infrarot Welle des erfindungsgemäßen Polymermaterials bewirkt.

In einer weiteren alternativ oder kumulativ bevorzugten Ausgestaltung kennzeichnet sich das erfindungsgemäße Polymermaterial dadurch, dass das oder mindestens eines der unterschiedlichen Dotierungselemente eine Größe im Bereich von 1 bis 10 nm aufweist und mit einem Abstand derart versehen ist, dass sich Elektronenwolken zweier Dotierungselemente mindestens bereichsweise überlappen. Mit anderen Worten, das erfindungsgemäße Polymermaterial weist das oder mindestens eines der unterschiedlichen Dotierungselemente zumindest abschnittsweise in einer solchen Verteilung im Polymerträger auf, dass aufgrund dieser Anordnung, insbesondere der bereichsweisen Überlappung der Elektronenwolken der unterschiedlichen Dotierungselemente eine kinetische Energie entsteht, die eine Trägerwelle des Polymermaterials hervorruft. Dies beruht auf der Elektronenwanderung und einem physikalischen Effekt, dem sogenannten "Skin Effekt", der im Bereich des Gleichstroms und Elektronen-Hoppings erweitert werden muss. Jeder Wechsel einer Laufrichtung erzeugt einen Impuls, den sogenannten "sound". Bei paralleler Schaltung addieren sich die Spannungen im Gegensatz zu einer seriellen Schaltung oder eines seriellen Elektronenlaufes.

Die erfindungsgemäßen alternativ oder kumulativ bevorzugten Ausgestaltungen des ersten Erfindungsgegenstandes können in jeder technisch sinnvollen Kombination realisiert werden. Hierbei können Merkmale aus den Ausführungsbeispielen einzeln oder in Kombination mit Merkmalen der detaillierten Beschreibung soweit technisch sinnvoll verwendet werden.

Gemäß zweitem Erfindungsgegenstand wird ein textiles Material umfassend ein erfindungsgemäßes Polymermaterial beansprucht. Die bevorzugten Ausgestaltungen hinsichtlich der Merkmale des erfindungsgemäßen Polymermaterials gemäß erstem Erfindungsgegenstand sind ebenfalls auf den vorliegenden zweiten Erfindungsgegenstand des textilen Materials anwendbar.

Gemäß einer bevorzugten Ausgestaltung wird das erfindungsgemäße textile Material ausgewählt aus der Gruppe bestehend aus Bekleidung, vorzugsweise Oberbekleidung, Unterbekleidung, Strümpfe umfassend Stützstrümpfe, T-Shirts, Langarmshirts, Hosen, insbesondere Laufhosen, Schuhe, insbesondere Schuhobermaterial, Innenfutter und Einlegesohle; Bettauflage; Bettbezug; Kissenbezug; Sitzbezug; und Verbandstoff zur Wundversorgung. Das erfindungsgemäße textile Material kann das erfindungsgemäße Polymermaterial in all seinen möglichen erfindungsgemäßen Ausgestaltungen umfassen, wobei das Polymermaterial beispielsweise zumindest teilweise als Polymerfaser verarbeitet, insbesondere verwoben, verstrickt, verwirkt oder verknüpft oder als Folie integriert ist oder die Oberfläche eines textilen Materials zumindest teilweise mit dem erfindungsgemäßen Polymermaterial beschichtet, insbesondere kaschiert ist.

Gemäß dem dritten Erfindungsgegenstand wird das erfindungsgemäße Polymermaterial zur Verwendung als Adjuvanz oder Prophylaktikum in therapeutischen Verfahren beansprucht. Dieser Erfindungsgegenstand umfasst ebenfalls alle Ausgestaltungen hinsichtlich der Merkmale des erfindungsgemäßen Polymermaterials gemäß erstem Erfindungsgegenstand. Gemäß vorliegender Erfindung wird demnach die erste Anwendung der entsprechenden Dotierungselemente in Polymermaterialien, insbesondere Eisenlegierungen, bevorzugt eine Eisenoxid-Legierung (Ferrite), weiter bevorzugt eine Erdalkali-Eisenoxid-Legierung (Erdalkali-Ferrite), insbesondere Barium Ferrite oder Strontium Ferrite, besonders bevorzugt eine SrFe₁₂O₁₉-Legierung (SrO(γ Fe₂O₃)₆) umfasst in Polymermaterialien in therapeutischen Verfahren gelehrt.

Gemäß dem vierten Erfindungsgegenstand werden die Anwendung des erfindungsgemäßen Polymermaterials in therapeutischen Verfahren spezialisiert, insbesondere auf die prophylaktische oder adjuvante Behandlung einer stoffwechselinadäquaten Mikrozirkulation des Blutes im Wirbeltier, vorzugsweise Menschen; die Behandlung von nekrotischen Gefäßen; bei der Wundheilung, beispielsweise bei Dekubitus, Diabetisches Fußsyndrom, Ulcus cruris, Verbrennungen und für die Wundbehandlung von vielen weiteren chronischen und sekundär heilenden Wunden; von Diabetes mellitus, insbesondere Diabetes mellitus Typ I und/oder Diabetes mellitus Typ II; von Krebserkrankungen; von Protein-bedingten Erkrankungen, beispielsweise Alzheimer Erkrankung, oder Demenzerkrankung; von Thrombozytenerkrankungen; von Erythrozytenerkrankungen; von immunologischen Erkrankungen, wie beispielsweise immunologische Hyperaktivität; von infektiösen Erkrankungen, beispielsweise Wundinfektionen; von neurologischen Erkrankungen, insbesondere soweit diese auf einer Erkrankung der Ummantelung der Nervenzellen und/oder Synapsen beruhen.

Gemäß einem fünften Erfindungsgegenstand wird das erfindungsgemäße Polymermaterial zur nicht-medizinischen, insbesondere sportlichen Leistungssteigerung eines Wirbeltiers, vorzugsweise Menschen beansprucht. Aufgrund der verbesserten Mikrozirkulation des Blutes und der damit verbesserten Versorgung der Zellen bzw. der Geweberegion beispielsweise mit Sauerstoff, Nährstoffen, Aminosäuren und Proteinen werden die Zell- bzw. Organfunktionen verbessert und damit die körperliche und geistige Leistungsfähigkeit, insbesondere die sportliche Leistungsfähigkeit erhöht.

Gemäß dem sechsten Erfindungsgegenstand wird ein Verfahren zur Herstellung eines erfindungsgemäßen Polymermaterials, wobei alle bevorzugten Ausgestaltungen des ersten Erfindungsgegenstandes auch auf das erfindungsgemäße Verfahren Anwendung finden. Das erfindungsgemäße Verfahren kennzeichnet sich dadurch, dass es folgende Schritte umfasst oder daraus besteht:
a. Bereitstellung eines geeigneten gelösten Polymers,
b. Bereitstellung eines geeigneten Dotierungselementes,
c. Verdampfen des in Schritt b) bereitgestellten Dotierungselementes mittels geeigneter Verfahren und Einbringen des verdampften Dotierungselementes in das in Schritt a) bereit gestellte gelöste Polymer und
d. Extrudieren des erfindungsgemäßen Polymermaterials im elektrischen Feld.

Das erfindungsgemäß zu verwendende Polymer gemäß aller Ausgestaltungen des ersten Erfindungsgegenstandes wird in geeigneten Lösungsmitteln in Lösung gebracht, so dass das verdampfte Dotierungselement in das gelöste Polymer eingebracht werden kann.

Das erfindungsgemäß zu verwendende Dotierungselement gemäß aller Ausgestaltungen des ersten Erfindungsgegenstandes wird vorzugsweise derart in das gelöste Polymer eingebracht, dass das Polymer die Dotierungselemente im extrudierten erfindungsgemäßen Polymermaterial zur Polymermaterialoberfläche isoliert. Vorzugsweise sind die Dotierungselemente im extrudierten erfindungsgemäßen Polymermaterial inhomogen verteilt, wobei die einzelnen Dotierungselemente in molekularer Größe, vorzugsweise im Bereich von 1 bis 10 nm vorliegen.

Gemäß einer alternativ oder kumulativ bevorzugten Ausgestaltung kennzeichnet sich das erfindungsgemäße Verfahren dadurch, dass in Schritt c) das Dotierungselement mittels einer geeigneten Verdampfungstechnologien vorzugsweise Plasma-Verdampfungs-Abscheidung (plasma vapor deposition, kurz PVD), insbesondere einer thermischen (Vakuum)-Verdampfungstechnik, Sputtern oder ähnlichen Techniken verdampft wird und in die Lösung des Polymers abgeschieden wird. Alternativ kann das Dotierungselement durch eine Magnetron-Verdampfung, beispielsweise durch Verwendung von Mikrowellenstrahlung, in die Gasphase überführt werden und so ein polymerbeschichtetes Plasma entstehen, indem man die Dotierungselemente und das Polymer in einem abgeschlossenen Raum schlagartig erhitzt. Bei dieser Methode variieren jedoch die Partikelgrößen sehr stark. Je kleiner die Polymermenge, in welche die verdampften Dotierungselemente eingebracht werden, desto weniger beeinflussen Schwere- und Trägheitsphänomene die Herstellung des erfindungsgemäßen Polymermaterials.

Gemäß einer alternativ oder kumulativ bevorzugten Ausgestaltung kennzeichnet sich das erfindungsgemäße Verfahren dadurch, dass in Schritt d) das Polymermaterial mittels einer geeigneten Extrusionstechnik, insbesondere durch Ausnutzung eines elektrischen Feldes, wie beispielsweise Elektro-Spinning Technologie, insbesondere Blow Spinning, extrudiert.

Gemäß einer alternativ oder kumulativ bevorzugten Ausgestaltung kennzeichnet sich das erfindungsgemäße Verfahren dadurch, dass eines oder mehrerer der Verfahrensschritte unter sterilen Bedingungen und/oder im Vakuum durchgeführt werden.

In einer weiteren alternativ oder kumulativ bevorzugten Ausgestaltung wird im erfindungsgemäßen Verfahren zur Herstellung eines Polymermaterials das Polymermaterial als geeignete Faser oder Folie extrudiert wird, die bei der Herstellung von erfindungsgemäßen textilen Materialien des zweiten Erfindungsgegenstandes verwendet werden können. Alternativ oder kumulativ kann das erfindungsgemäße Polymermaterial so extrudiert werden, dass es zumindest Teile des textilen Material, vorzugsweise solche Teile die mit dem Körper eines Wirbeltiers, vorzugsweise Menschen in (direktem) Kontakt stehen, beschichtet. Die Beschichtung von textilem Trägermaterial wird vorzugsweise insbesondere bei textilen Materialien aus dem Bereich der Wundversorgung (Wundauflagen, Pflaster, OP-Abdeckungen etc.) angewendet.

Die vorliegende Erfindung wird im Weiteren anhand von beispielhaften Ausführungsarten beschrieben, die lediglich als Beispiele verstanden werden sollen und die nicht den Schutzumfang des vorliegenden Schutzrechtes auf diese Ausführungsformen beschränken sollen. Die einzelnen Merkmale der nachfolgenden Ausführungsbeispiele können separat oder in (Teil-)Kombinationen vorzugsweise verwendet werden.

### AUSFÜHRUNGSBEISPIELE:

### A: Herstellung eines erfindungsgemäßen Polymermaterials

Die erfindungsgemäßen Dotierungselemente, beispielsweise eine SrFe₁₂O₁₉-Legierung (SrO(γ Fe₂O₃)₆) werden Metall pyrolytisch verdampft, vorzugsweise mittels Plasma-Vakuum-Verdampfungstechnologie. Das entstehende Gas wird durch Injektion in eine geeignete Menge gelösten Polymers, vorzugsweise eines Polyesters, insbesondere Polyethylenterephthalat, oder eines Polyamids eingebracht. Durch Anlegen eines elektrischen Feldes wird beispielsweise eine erfindungsgemäße Polymermaterialfaser gesponnen. Hierfür wird vorzugsweise ein E-Spinning-Verfahren verwendet, wobei ein Draht durch einen gelösten Tropfen Polymer gezogen wird, in den das verdampfte Dotierungselement eingebracht ist. Durchmesser und Länge des Drahtes werden bestimmt durch den Abstand des Magneten zum gelösten Polymer und der Stärke des elektrischen Feldes.

Das Gewichtsverhältnis von Dotierungselement und Polymer beträgt im resultierenden erfindungsgemäßen Polymermaterial, das als Faser versponnen wurde 4:6.

### B: Herstellung eines erfindungsgemäßen textilen Materials sowie einer Kontrolle

### B.1: Kontroll-Schuheinlage für die Placebokontrolle

Für die nachfolgend beschriebene doppel-verblindete placebokontrollierte Untersuchung wurde eine kommerziell erhältliche Standard-Schuheinlage der Firma ECCO verwendet, die kein erfindungsgemäßes Polymermaterial als Absorberschicht enthält und deren Obermaterial nachgelocht wurde, um der erfindungsgemäßen Schuheinlage im Aufbau zu entsprechen.

### B.2: Herstellung einer erfindungsgemäßen Schuheinlage

Für die nachfolgend beschriebene doppel-verblindete placebokontrollierte Untersuchung wird eine erfindungsgemäße Schuheinlage verwendet, die im Aufbau mit der vorgenannten Kontroll-Schuheinlage vergleichbar ist, wobei die Absorberschicht das erfindungsgemäße Polymermaterial (vgl. Beispiel A) enthält und von dem gelochten Obermaterial abdeckt ist (vgl. Kontrollschuheinlage gemäß B1).

### C: Placebokontrollierte Untersuchungen zur Beeinflussung der Mikrozirkulation

### C.1: Design der Untersuchungen

Untersucht wurde eine Gesamtstichprobe N_{ges} = 72 Probanden, unterteilt in 4 hinreichend homogene Teilstichproben je n = 18 (jeweils mit 9 männlichen und 9 weiblichen Probanden).

**Tabelle 1: Darstellung der Teilstichproben, Probanden- bzw. Patientengut und Alter der placebokontrollierten Untersuchung:**

| **Teilstichproben** | **Probanden-bzw. Patientengut** | **Alter** |
|---|---|---|
| A | Gesunde untrainierte Probanden | ≈ 25 Jahre |
| B | Gesunde trainierte Probanden | ≈ 25 Jahre |
| C | Ältere Rehabilitanden (physische Konditionierung) | ≈ 54 Jahre |
| D | Ältere Diabetiker (Diabetes mellitus Typ II, eingestellt) | ≈ 55 Jahre |

GCP-konforme Ein- und Ausschlußkriterien.

Die Untersuchungen erfolgten verblindet. Jeder Proband in jeder Teilstichprobe nahm an zwei Untersuchungsreihen teil: Anwendung einer Placebo-Schuheinlage (Kontrolle) und Anwendung der erfindungsgemäßen Schuheinlage (Verum). Zwischen beiden Untersuchungsreihen lag ein Zeitintervall von 2 bis 3 Wochen. Die Reihenfolge der Teilnahme jedes Probanden an der Untersuchungsreihe Kontrolle oder der Untersuchungsreihe Verum wurde durch einen Zufallsgenerator festgelegt.

Die Untersuchungen erfolgten unter definierter körperlicher Betätigung der Probanden auf einem Laufband mit definierter Geschwindigkeit, welche einem leicht beschleunigten Spazierengehen entsprach. Die Laufbandbelastung erfolgte im Untersuchungszeitraum von 30 Tagen täglich in einem Zeitintervall von 60 Minuten bei einer Laufbandneigung von 5%, einer mittleren Laufbandgeschwindigkeit von 0,8 bis 1,0 m/s beginnend mit geringer Laufbandgeschwindigkeit und Steigerung im Verlauf der 60-minütigen Behandlung in Stufen alle 10 Minuten um 0,1 bis 0,2 m/s.

Als Untersuchungsmethode diente ein nicht-invasives hochauflösendes Meßverfahren auf dem neuesten Stand von Wissenschaft und Technik, die kombinierte Weißlicht-Spektroskopie und LaserDOPPLER-Mikroflußmessung (System LEA, Deutschland).

Gemessen wurde in einem repräsentativen Targetgewebe zeitgleich in zwei Gewebetiefen: 2mm und 8 mm. Als definiertes Targetgewebe der Messungen wurden die Subkutis und die Skelettmuskulatur in der linken Wade ausgewählt. Gemessen wurde am jeweiligen Behandlungstag unmittelbar vor Beginn der Belastung (Ausgangswerte), während der Belastung und unmittelbar nach dem Ende der 60 minütigen Laufbandbelastung. Die Erhebung der Meßwerte wird unter konstanten Randbedingungen vorgenommen, bei bequemen Sitzen unter konstanten makrozirkulatorischen und temperaturregulatorischen Randbedingungen. Zwei Stunden vor den Untersuchungen kein Alkohol, kein Kaffee, Tee oder Cola-Getränk. Mindestens 6 Stunden Schlaf täglich, keine biotrope Wetterlage im Beobachtungsintervall.

Folgende Merkmale des Funktionszustandes der Mikrozirkulation wurden insbesondere ermittelt:
- Strömungsfluß der roten Blutzellen in den Mikrogefäßen Q_{RBC}
- Mittlere Strömungsgeschwindigkeiten der roten Blutzellen in den Mikrogefäß-Netzwerken V_{Rsc}
- Relative Hämoglobin-Sättigung in den Mikrogefäßen rHb
- Anzahl der blutzellperfundierten Knotenpunkte im definierten Netzwerk nNP
- Venolenseitige Sauerstoffausschöpfung in der Mikrozirkulation des Targetgewebes ΔPO₂

### Meßzeitpunkte:

Am 0. Tag wurden die Ausgangswerte gemessen, an den Tagen 1. bis 30 wurden täglich Messungen vor und nach Laufbandbelastung vorgenommen.

Zur statistischen Auswertung der erhaltenen Meßdaten diente ein parameterfreies Prüfverfahren mit hoher Trennschärfe, der WILCOXON-Rangsummentest auf dem Signifikanzniveau alpha = 5%.

### C.2: Untersuchungsergebnisse

Von herausragender Bedeutung für eine medizinische Bewertung des therapeutischen Erfolges der geprüften speziellen Schuheinlage auf den Funktionszustand der Mikrozirkulation sind die Merkmale mittlerer Strömungsfluß der roten Blutzellen in den Mikrogefäßen Q_{RBC}, Anzahl der blutzellperfundierten Knotenpunkte im Mikrogefäßnetzwerk nNP und venolenseitige Sauerstoffausschöpfung ΔPO₂ im aktiven Muskelgewebe.

### C.2.1: Merkmal mittlerer Strömungsfluß der roten Blutzellen in den Mikrogefäßen Q_{RBC}

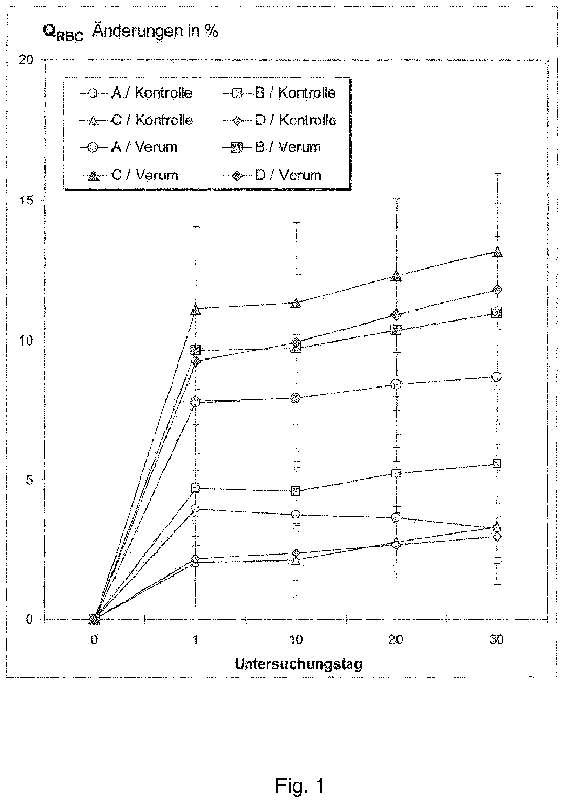

Figur 1 zeigt die Meßdaten zum Merkmal mittlerer Strömungsfluss der roten Blutzellen in den mikrovaskulären Netzwerken der Skelettmuskulatur Q_{RBC} in der linken Wade des Probanden / Patienten (Mittelwerte und Standardabweichungen) bei den Teilstichproben A, B, C und D für Kontrolle und Verum (Mittelwerte und Standardabweichungen). Ordinate: Merkmaländerungen in Prozent (im Vergleich mit den Ausgangswerten). Abszisse: Meßtage im 30-tägigen Untersuchungszeitraum.

Je nach Alter und körperlicher Konstitution der Probanden bzw. Patienten treten unterschiedliche Merkmaländerungen auf. Die Merkmaländerungen in den Placebo-Gruppen (Kontrollen) erreichen maximal - 5 % am 0. Tag im Vergleich mit ihren jeweiligen Ausgangswerten zum Zeitpunkt t = 0. Nach Anwendung der erfindungsgemäßen Schuheinlage (Verum-Gruppen) treten deutlich höhere Beträge der Merkmaländerungen auf, die z.T. mehr als doppelt so hohe Werte erreichen.

### C.2.2: Merkmal Anzahl der blutzellperfundierten Knotenpunkte im Mikrogefäßnetzwerk nNP

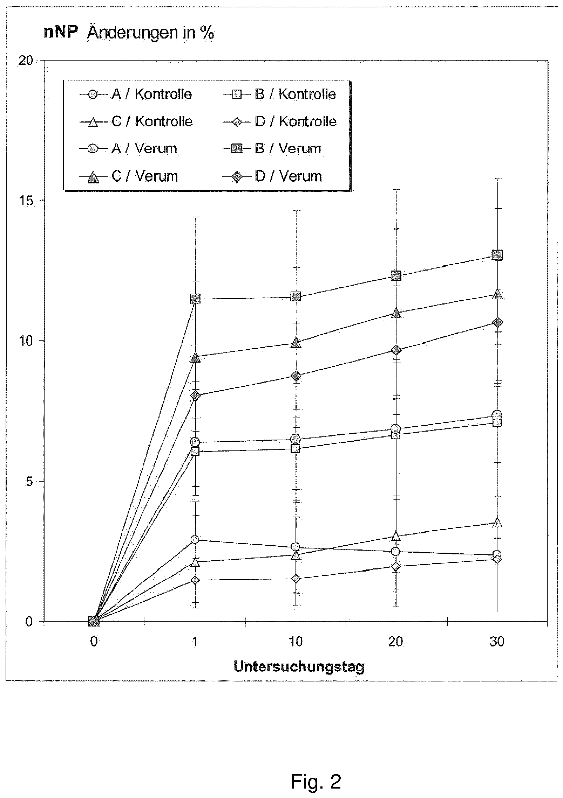

Figur 2 zeigt die Meßdaten zum Merkmal Anzahl der blutzellperfundierten Knotenpunkte im Mikrogefäßnetzwerk nNP in der linken Wade des Probanden / Patienten (Mittelwerte und Standardabweichungen) bei den Teilstichproben A, B, C und D für Kontrolle und Verum (Mittelwerte und Standardabweichungen). Ordinate: Merkmaländerungen in Prozent (im Vergleich mit den Ausgangswerten). Abszisse: Meßtage im 30-tägigen Untersuchungszeitraum.

Die Merkmaländerungen zu nNP lassen ein korrespondierendes Verhalten zu den Merkmaländerungen von Q_{RBC} erkennen. Mit anderen Worten: Je nach Alter und körperlicher Konstitution der Probanden bzw. Patienten treten unterschiedliche Merkmaländerungen auf, wobei auch bei diesem Merkmal nach Anwendung der erfindungsgemäßen Schuheinlage (Verum-Gruppe) deutlich höhere Beträge der Merkmaländerungen auftreten, die z.T. mehr als doppelt so hohe Werte erreichen.

### C.2.3: Merkmal venolenseitige Sauerstoffausschöpfung ΔPO₂

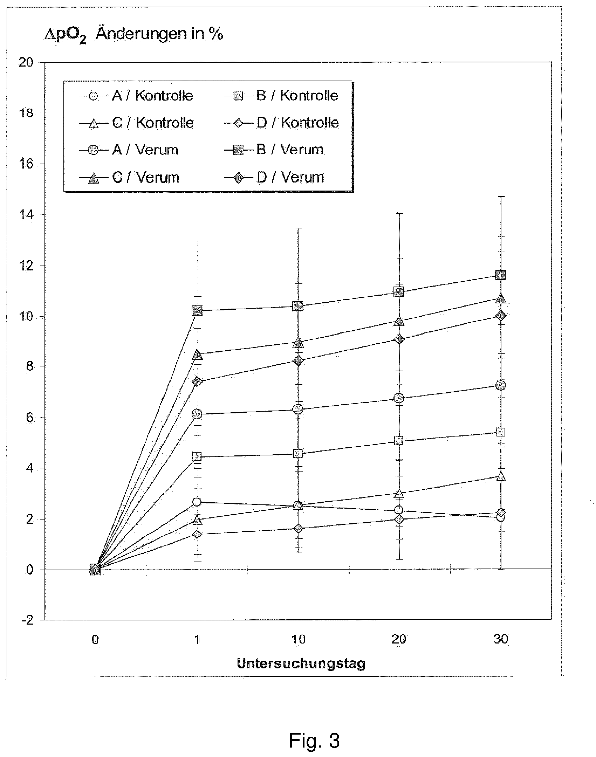

Figur 3 zeigt die Meßdaten zum Merkmal venolenseitige Sauerstoffausschöpfung in den mikrovaskulären Netzwerken der Skelettmuskulatur ΔPO₂ in der linken Wade des Probanden / Patienten (Mittelwerte und Standardabweichungen) bei den Teilstichproben A, B, C und D für Kontrolle und Verum (Mittelwerte und Standardabweichungen). Ordinate: Merkmaländerungen in Prozent (im Vergleich mit den Ausgangswerten). Abszisse: Meßtage im 30-tägigen Untersuchungszeitraum.

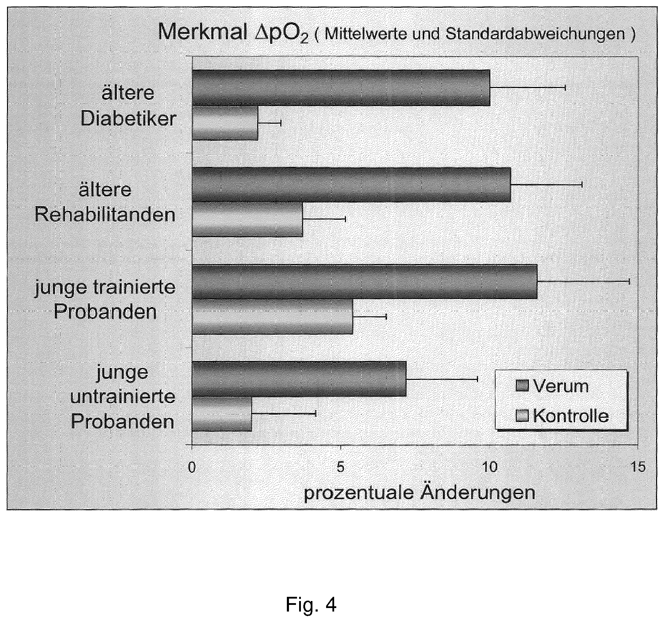

In Figur 4 sind die Differenzbeträge der Merkmaländerungen zur venolenseitigen Sauerstoffausschöpfung ΔPO₂ in den mikrovaskulären Netzwerken der Skelettmuskulatur der linken Wade des Probanden / Patienten Mittelwerte und Standardabweichungen) der Ausgangswerte am 0. Tag zu den Meßwerten am 30. Tag bei den Teilstichproben A, B, C und D für Kontrolle und Verum (Mittelwerte und Standardabweichungen) als Säulendiagramm dargestellt.

Die Merkmaländerungen zu ΔPO₂ lassen ein korrespondierendes Verhalten zu den Merkmaländerungen von Q_{RBC} und/oder ΔPO₂ erkennen. Mit anderen Worten: Je nach Alter und körperlicher Konstitution der Probanden bzw. Patienten treten unterschiedliche Merkmaländerungen auf, wobei auch bei diesem Merkmal nach Anwendung der erfindungsgemäßen Schuheinlage (Verum-Gruppe) deutlich höhere Beträge der Merkmaländerungen auftreten, die insbesondere zwischen 2- und 5-fach so hohe Werte erreichen.

Sowohl bei jüngeren als auch bei den älteren Probanden bzw. Patienten zeigt sich ein therapeutischer Erfolg der erfindungsgemäßen Schuheinlage. Die größten Unterschiede zwischen Kontrolle (Placebo) und Verum (erfindungsgemäße Schuheinlage) wurden bei älteren Diabetikern und älteren Rehabilitanden festgestellt.

**Tabelle 2: Prozentuale Änderungen der venolenseitigen Sauerstoffausschöpfung ΔPO₂ in der Skelettmuskulatur der linken Wade am 30. Tag im Vergleich mit den jeweiligen Ausgangswerten am 0. Tag (Mittelwerte und Standardabweichungen):**

| **Teilstichproben** | **Kontrolle (Placebo)** | **Erfindungsgemäße Schuheinlage (Verum)** |
|---|---|---|
| A: gesunde untrainierte Probanden (Alter ca. 25 Jahre) | 2,0 (2,16) | 7,2 (2,41) |
| B: gesunde trainierte Probanden (Alter ca. 25 Jahre) | 5,4 (1,13) | 11,6 (3,11) |
| C: ältere Rehabilitanden (Alter ca. 54 Jahre) | 3,7 (1,46) | 10.7 (2,41) |
| D: ältere Diabetiker (Alter ca. 55 Jahre) | 2,2 (0,77) | 10,0 (2,54) |

Aus den Untersuchungsergebnissen gemäß Figuren 1 bis 4 lässt sich zudem ableiten, dass zwischen dem Verhalten der Merkmale Q_{RBC} und nNP sowie dem Merkmal venolenseitige Sauerstoffausschöpfung ΔPO₂ ein funktioneller Zusammenhang besteht.

### C.2.4: Vitalmikroskopisches Befundbeispiel von einem älteren Diabetiker

### (D), Verum (erfindungsgemäße Schuheinlage)

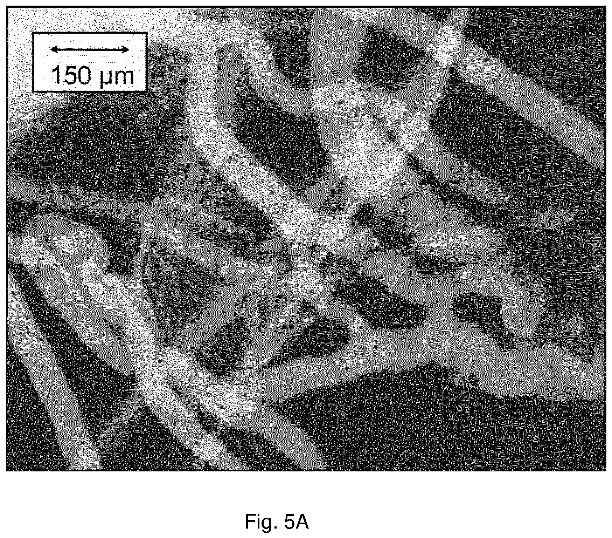

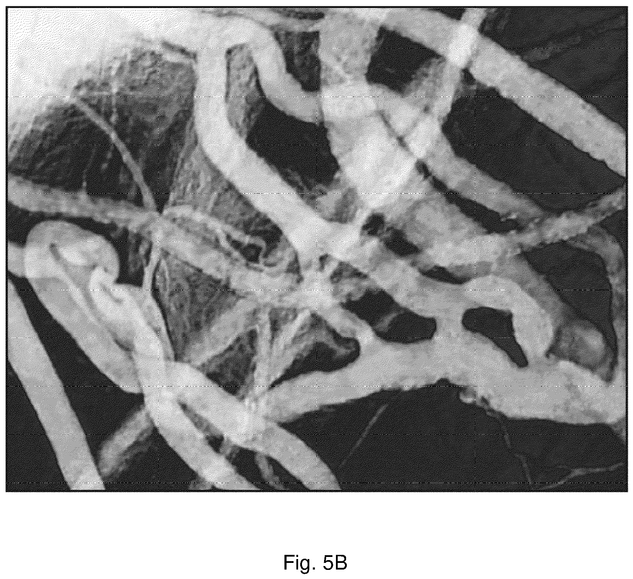

Figuren 5A und 5B zeigen vitalmikroskopische Befundbeispiele eines älteren Diabetiker (Teilstichprobe D), der Verum-Gruppe, vor Durchführung der Untersuchung (Fig. 5A) und nach Anwendung der erfindungsgemäßen Schuheinlage am 30. Tag der Untersuchung (5B). Gezeigt wird ein Bereich der Subkutis der linken Wade (Kapillaren, Arteriolen, Venolen).

In den Figuren 5A und 5B sind mittels Pseudofarbtransformation der Primärabbildung die blutzellperfundierten Mikrogefäße GELB markiert, was einem hellem grau in einer Schwarz-Weiß-Darstellung entspricht.

Der Verteilungszustand des Plasma-Blutzell-Gemisches in der gleichen Geweberegion wird zu zwei verschiedenen Beobachtungszeitpunkten gezeigt:
Vorher (Ausgangsbedingung am 0. Tag)
Nachher (Verteilungszustand am 30. Tag nach Anwendung der erfindungsgemäßen Schuheinlage)

Aus den Abbildungen der Figuren 5A und 5B erkennt man die deutliche Zunahme blutzellperfundierter Mikrogefäße und damit eine Erweiterung der mikrozirkulatorischen Reserve.

### C.2.5: Mittlere Strömungsgeschwindigkeiten der roten Blutzellen in den Mikrogefäß-Netzwerken V_{Rsc} und Relative Hämoglobin-Sättigung in den Mikrogefäßen rHb

Die Meßdaten zum Merkmal mittlere Strömungsgeschwindigkeiten der roten Blutzellen in den mikrovaskulären Netzwerken der Skelettmuskulatur V_{Rsc} in der linken Wade des Probanden / Patienten bei den Teilstichproben A, B, C und D für Kontrolle und Verum zeigen überraschenderweise, dass die roten Blutzellen bis um den Faktor 1,4 schneller als das Plasma strömen.

Die Meßdaten zum Merkmal relative Hämoglobin-Sättigung in den Mikrogefäßen rHb in der linken Wade des Probanden / Patienten bei den Teilstichproben A, B, C und D für Kontrolle und Verum zeigen überraschenderweise eine kurzfristige Verdopplung der relativen Hämoglobinsättigung rHb.

Mit anderen Worten, die roten Blutzellen sind in der Verum-Gruppe schneller und röter als die roten Blutzellen der Kontroll-Gruppe.

Weitere Ausführungsbeispiele:
1. Polymermaterial umfassend ein oder mehrere unterschiedliche Dotierungselemente, dadurch gekennzeichnet, dass das oder mindestens eines der unterschiedlichen Dotierungselemente zumindest teilweise eine vom Wirbeltier, vorzugsweise Menschen emittierte elektromagnetische Strahlung absorbieren und zumindest teilweise eine elektromagnetische Strahlung im Infrarot Bereich emittieren.
2. Polymermaterial gemäß Ausführungsbeispiel 1, dadurch gekennzeichnet, dass das Polymer ausgewählt wird aus der Liste bestehend aus der Gruppe der Polyester, beispielsweise Polyethylenterephthalat (PET), der Gruppe der Polyamide, beispielsweise Poly(p-phenylenterephthalamid) (PPTA) und Poly(*m*-phenylenisophthalamid) (PMPI).
3. Polymermaterial gemäß Ausführungsbeispiel 1 oder 2, dadurch gekennzeichnet, dass das oder mindestens eines der unterschiedlichen Dotierungselemente eine Eisen Legierung umfasst, bevorzugt eine Eisenoxid-Legierung, weiter bevorzugt eine Erdalkali-Eisenoxid-Legierung, besonders bevorzugt eine SrFe₁₂0₁₉ - Legierung.
4. Polymermaterial gemäß einem der Ausführungsbeispiele 1 bis 3, dadurch gekennzeichnet, dass das oder die unterschiedlichen Dotierungselemente in das Innere des Polymermaterials eingebracht sind und das Polymer die Dotierungselemente zur Polymermaterialoberfläche isoliert.
5. Polymermaterial gemäß einem der Ausführungsbeispiele 1 bis 4, dadurch gekennzeichnet, dass das oder mindestens eines der unterschiedlichen Dotierungselemente eine Größe im Bereich von 1 bis 10 nm aufweist und mit einem Abstand zueinander derart versehen ist, dass Elektronenwolken zweier Dotierungselemente mindestens bereichsweise überlappen.
6. Polymermaterial gemäß einem der Ausführungsbeispiele 1 bis 5, dadurch gekennzeichnet, dass die emittierte Infrarot Strahlung eine Wellenlänge im Bereich von 3 bis 15 µm aufweist.
7. Textiles Material umfassend ein Polymermaterial gemäß einem der Ausführungsbeispiele 1 bis 6.
8. Textiles Material gemäß Ausführungsbeispiel 7, dadurch gekennzeichnet, dass das textile Material ausgewählt wird aus der Gruppe bestehend aus Bekleidung, vorzugsweise Oberbekleidung, Unterbekleidung, Strümpfe umfassend Stützstrümpfe, T-Shirts, Langarmshirts, Hosen, insbesondere Laufhosen, Schuhe, insbesondere Schuhobermaterial, Innenfutter und Einlegesohle; Bettauflage; Bettbezug; Kissenbezug; Sitzbezug; und Verbandstoff zur Wundversorgung.
9. Polymermaterial gemäß einem der Ausführungsbeispiele 1 bis 6 zur Verwendung als Adjuvanz oder Prophylaktikum in therapeutischen Verfahren.
10. Polymermaterial gemäß einem der Ausführungsbeispiele 1 bis 6 zur Verwendung bei der prophylaktischen oder adjuvanten Behandlung einer stoffwechselinadäquaten Mikrozirkulation des Blutes im Wirbeltier, vorzugsweise Menschen; bei der Behandlung von nekrotischen Gefäßen; bei der Wundheilung; von Diabetes mellitus; von Krebserkrankungen; von Protein-bedingten Erkrankungen; von Thrombozytenerkrankungen; von Erythrozytenerkrankungen; von immunologischen Erkrankungen; von infektiösen Erkrankungen; von neurologischen Erkrankungen, insbesondere soweit diese auf einer Erkrankung der Ummantelung der Nervenzellen und/oder Synapsen beruhen.
11. Verwendung eines Polymermaterials gemäß einem der Ausführungsbeispiele 1 bis 6 zur nicht-medizinischen, insbesondere sportlichen Leistungssteigerung eines Wirbeltiers, vorzugsweise eines Menschen.
12. Verfahren zur Herstellung eines Polymermaterials gemäß einem der Ausführungsbeispiele 1 bis 6, dadurch gekennzeichnet, dass das Verfahren folgende Schritte umfasst oder daraus besteht: a. Bereitstellung eines geeigneten gelösten Polymers, b. Bereitstellung eines geeigneten Dotierungselementes, c. Verdampfen des in Schritt b) bereitgestellten Dotierungselementes mittels geeigneter Verfahren und Einbringen des verdampften Dotierungselementes in das in Schritt a) bereit gestellte gelöste Polymer und Extrudieren des Polymermaterials gemäß einem der Ausführungsbeispiele 1 bis 6 im elektrischen Feld.
13. Verfahren zur Herstellung eines Polymermaterials gemäß Ausführungsbeispiel 12, dadurch gekennzeichnet, dass in Schritt c) das Dotierungselement mittels Plasma-Verdampfungstechnologie (PVD) verdampft wird und in die Lösung des Polymers abgeschieden wird und/oder dass in Schritt d) das Polymermaterial mittels Elektro-Spinning Technologie extrudiert wird und/oder dass eines oder mehrerer der Verfahrensschritte unter sterilen Bedingungen und/oder im Vakuum durchgeführt werden.
14. Verfahren zur Herstellung eines Polymermaterials gemäß Ausführungsbeispiel 12 oder 13, dadurch gekennzeichnet, dass die Dotierungselemente in der Größe von 1 bis 10 nm in das Polymermaterial eingebettet sind und das Polymermaterial die Dotierungselemente zur Polymermaterialoberfläche isoliert.
15. Verfahren zur Herstellung eines Polymermaterials gemäß einem der Ausführungsbeispiele 12 bis 14, dadurch gekennzeichnet, dass das Polymermaterial als Faser oder Folie extrudiert wird oder dass weitere textile Materialien zumindest teilweise mit dem Polymermaterial beschichtet werden.

## Patentansprüche

1. Polymermaterial umfassend ein oder mehrere unterschiedliche Dotierungselemente, **dadurch gekennzeichnet, dass**
- die Dotierungselemente im Verhältnis zum Polymermaterial <1 : 1 Gewichtsanteile umfassen,
- das oder die unterschiedlichen Dotierungselemente in das Innere des Polymermaterials eingebracht sind und das Polymer die Dotierungselemente zur Polymermaterialoberfläche isoliert,
- das oder mindestens eines der unterschiedlichen Dotierungselemente zumindest teilweise eine vom Wirbeltier, vorzugsweise Menschen emittierte elektromagnetische Strahlung absorbieren und zumindest teilweise eine elektromagnetische Strahlung im Infrarot Bereich mit einer Wellenlänge im Bereich von 3 µm bis 50 µm emittieren, und
- die Dotierungselemente eine Größe im Bereich von 1 bis 10 nm aufweisen und einen Abstand zueinander aufweisen, dass Elektronenwolken zweier Dotierungselemente mindestens bereichsweise überlappen so dass bei der Absorption der elektromagnetischen Strahlung durch das Polymermaterial eine kinetische Energie entsteht, die eine Trägerwelle des Polymermaterials im Bereich von mehr als 60 Dezibel, vorzugsweise 60 bis 130 Dezibel hervorruft, wodurch die vom Polymermaterial emittierte Strahlung im Infrarot-Bereich beschleunigt wird.

2. Polymermaterial gemäß Anspruch 1, **dadurch gekennzeichnet, dass** das Polymer ausgewählt wird aus der Liste bestehend aus der Gruppe der Polyester, beispielsweise Polyethylenterephthalat (PET), der Gruppe der Polyamide, beispielsweise Poly(*p*-phenylenterephthalamid) (PPTA) und Poly(m-phenylenisophthalamid) (PMPI).

3. Polymermaterial gemäß Anspruch 1 oder 2, **dadurch gekennzeichnet, dass** das oder mindestens eines der unterschiedlichen Dotierungselemente eine Eisen Legierung umfasst, bevorzugt eine Eisenoxid-Legierung, weiter bevorzugt eine Erdalkali-Eisenoxid-Legierung, besonders bevorzugt eine SrFe₁₂O₁₉-Legierung.

4. Textiles Material umfassend ein Polymermaterial gemäß einem der Ansprüche 1 bis 3.

5. Textiles Material gemäß Anspruch 4, **dadurch gekennzeichnet, dass** das textile Material ausgewählt wird aus der Gruppe bestehend aus Bekleidung, vorzugsweise Oberbekleidung, Unterbekleidung, Strümpfe umfassend Stützstrümpfe, T-Shirts, Langarmshirts, Hosen, insbesondere Laufhosen, Schuhe, insbesondere Schuhobermaterial, Innenfutter und Einlegesohle; Bettauflage; Bettbezug; Kissenbezug; Sitzbezug; und Verbandstoff zur Wundversorgung.

6. Polymermaterial gemäß einem der Ansprüche 1 bis 4 zur Verwendung bei der prophylaktischen oder adjuvanten Behandlung einer stoffwechselinadäquaten Mikrozirkulation des Blutes im Wirbeltier, vorzugsweise Menschen; bei der Behandlung von nekrotischen Gefäßen; bei der Wundheilung; von Diabetes mellitus; von Krebserkrankungen; von Protein-bedingten Erkrankungen; von Thrombozytenerkrankungen; von Erythrozytenerkrankungen; von immunologischen Erkrankungen; von infektiösen Erkrankungen; von neurologischen Erkrankungen, insbesondere soweit diese auf einer Erkrankung der Ummantelung der Nervenzellen und/oder Synapsen beruhen.

7. Verfahren zur Herstellung eines Polymermaterials gemäß einem der Ansprüche 1 bis 4, **dadurch gekennzeichnet, dass** das Verfahren folgende Schritte umfasst oder daraus besteht:
a. Bereitstellung eines geeigneten gelösten Polymers,
b. Bereitstellung eines geeigneten Dotierungselementes mit einer Größe im Bereich von 1 bis 10 nm,
c. Verdampfen des in Schritt b) bereitgestellten Dotierungselementes mittels geeigneter Verfahren und Einbringen des verdampften Dotierungselementes in das in Schritt a) bereit gestellte gelöste Polymer und
d. Extrudieren des Polymermaterials im elektrischen Feld, so dass im extrudierten Polymermaterial
- die Dotierungselemente im Verhältnis zum Polymermaterial <1 : 1 Gewichtsanteile umfassen,
- das oder die unterschiedlichen Dotierungselemente in das Innere des Polymermaterials eingebracht sind und das Polymer die Dotierungselemente zur Polymermaterialoberfläche isoliert,
- das oder mindestens eines der unterschiedlichen Dotierungselemente zumindest teilweise eine vom Wirbeltier, vorzugsweise Menschen emittierte elektromagnetische Strahlung absorbieren und zumindest teilweise eine elektromagnetische Strahlung im Infrarot Bereich mit einer Wellenlänge im Bereich von 3 µm bis 50 µm emittieren, und
- die Dotierungselemente eine Größe im Bereich von 1 bis 10 nm aufweisen und einen Abstand zueinander aufweisen, dass Elektronenwolken zweier Dotierungselemente mindestens bereichsweise überlappen so dass bei der Absorption der elektromagnetischen Strahlung durch das Polymermaterial eine kinetische Energie entsteht, die eine Trägerwelle des Polymermaterials im Bereich von mehr als 60 Dezibel, vorzugsweise 60 bis 130 Dezibel hervorruft, wodurch die vom Polymermaterial emittierte Strahlung im Infrarot-Bereich beschleunigt wird.

8. Verfahren zur Herstellung eines Polymermaterials gemäß Anspruch 7, **dadurch gekennzeichnet, dass** in Schritt c) das Dotierungselement mittels Plasma-Verdampfungstechnologie (PVD) verdampft wird und in die Lösung des Polymers abgeschieden wird und/oder dass in Schritt d) das Polymermaterial mittels Elektro-Spinning Technologie extrudiert wird und/oder dass eines oder mehrerer der Verfahrensschritte unter sterilen Bedingungen und/oder im Vakuum durchgeführt werden.

9. Verfahren zur Herstellung eines Polymermaterials gemäß Anspruch 7 oder 8, **dadurch gekennzeichnet, dass** das Polymermaterial als Faser oder Folie extrudiert wird oder dass weitere textile Materialien zumindest teilweise mit dem Polymermaterial beschichtet werden.
